Europäisches Patentamt

European Patent Office

Office européen des brevets

(11) Publication number: **0 253 870 B1**

# EUROPEAN PATENT SPECIFICATION

(45) Date of publication of patent specification: **31.03.93**  (51) Int. Cl.5: **C12P 21/02**

(21) Application number: **87900933.0**

(22) Date of filing: **02.01.87**

(86) International application number:
**PCT/US87/00033**

(87) International publication number:
**WO 87/04187 (16.07.87 87/15)**

The file contains technical information submitted
after the application was filed and not included in
this specification

(54) **METHOD FOR PRODUCING FACTOR VIII:c-TYPE PROTEINS.**

(30) Priority: **03.01.86 US 816031**
**16.12.86 US 942338**

(43) Date of publication of application:
**27.01.88 Bulletin 88/04**

(45) Publication of the grant of the patent:
**31.03.93 Bulletin 93/13**

(84) Designated Contracting States:
**AT BE CH DE FR GB IT LI LU NL SE**

(56) References cited:
**EP-A- 0 112 174     EP-A- 0 251 843**
**EP-A- 0 254 076     WO-A-85/01961**
**GB-A- 2 129 685     JP-A- 0 013 389**
**JP-A- 0 023 784     JP-A- 0 166 079**
**JP-A- 0 173 078**

(73) Proprietor: **GENETICS INSTITUTE, INC.**
**87 Cambridge Park Drive**
**Cambridge, Massachusetts 02140(US)**

(72) Inventor: **KAUFMAN, Randal, J.**
**111 Marlborough Street 1**
**Boston, MA 02116(US)**
Inventor: **ADAMSON, S., Robert**
**5 Taft Avenue**
**Lexington, MA 02173(US)**

(74) Representative: **Votier, Sidney David et al**
**CARPMAELS & RANSFORD 43, Bloomsbury**
**Square**
**London WC1A 2RA (GB)**

BRITISH JOURNAL OF HAEMATOLOGY, vol. 52, 1982, pages 259-267, Blackwell Scientific Publications; E.G.D. TUDDENHAM et al.: "Response to infusions of polyelectrolyte fractionated human factor VIII concentrate in human haemophilia A and von Willebrand's disease"

Nature 312, 22 November 1984, Wood et al "Expression of active human factor VIII from recombinant DNA clones", pages 330-337

Journal of clinical investigation 60, August 1977, Weiss et al "Stabilization of factor VIII in plasma by the von Willebrand factor", pages 390-404

Biochemical Journal 200, 1981, Anderson et al "Interaction of factor VIII - von Willebrand factor with phospholipid vesicles", pages 161-167

## Description

Background of the Invention

The Factor VIII complex has two distinct biologic functions: coagulant activity and a role in primary hemostatis. The analysis of Factor VIII deficiency diseases, classic hemophilia and von Willebrand's disease, have contributed to the understanding that Factor VIII is a complex of two components. The Factor VIII:c procoagulant protein (antihemophilic factor) and the Factor VIII related antigen (von Willebrand factor,VWF) are under separate genetic control, have distinct biochemical and immunologic properties, and have unique and essential physiologic functions.

The Factor VIII:c molecule is an important regulatory protein in the blood coagulation cascade. After activation by thrombin, it accelerates the rate of Factor X activation by Factor IXa, eventually leading to the formation of the fibrin clot. Deficiency of Factor VIII:c (classic hemophilia) is an X-linked chromosomal disorder that has been a major source of hemorrhagic morbidity and mortality in affected males. Treatment usually consists of frequent transfusions with blood products. The latter has resulted in a high incidence of infectious complications (such as various forms of hepatitis and acquired immunodeficiency disease) in the hemophiliac population.

The VWF molecule is an adhesive glycoprotein that plays a central role in platelet agglutination. It serves as a carrier for Factor VIII:c in plasma and facilitates platelet-vessel wall interactions. Discrete domains of VWF which bind to platelet receptor sites on glycoprotein 1b and on the glycoprotein IIb-IIIa complex, as well as binding sites on collagen have been noted. VWF is made up of multiple, probably identical, subunits each of 230,000 daltons. VWF is synthesized in endothelial cells and megakaryocytes. In the plasma it exists as high molecular weight multimers ranging from $5 \times 10^5$ to $10^7$ daltons. The Von Willebrand factor contains 5-6% complex carbohydrate, which appears important in the molecules ability to bind platelets. A variety of abnormalities in VWF activity can result in Von Willebrand's disease. The disorder is generally inherited in autosomal dominant fashion and may affect as many as one in 2000 individuals. Mild forms of the disease frequently go undiagnosed, whereas severely affected patients may require frequent blood product support with its associated risks.

Recently, the isolation of the genes for both factor VIII:c and Von Willebrand factor have made possible the production of recombinant factor VIII:c and VWF preparations which are essentially free of contaminating viruses (Toole et al. 1984, Wood et al. 1984, Lynch et al. Cell 41:49-56 1985, Ginsberg et al. Science 228:1401-1406 1985). The production of Factor VIII:c and analogs thereof through recombinant DNA technology has been achieved utilizing mammalian cells as a recipient of DNA encoding Factor VIII:c (or analogs thereof) contained in appropriate expression vectors. Primary concerns for the synthesis of a recombinant Factor VIII:c include (i) the yield of recombinant FVIII:c obtainable from the culture medium, (ii) the stability of the recombinant FVIII:c so produced, (iii) the efficiency and cost of purification of the FVIII:c and (iv) the overall cost of producing purified recombinant FVIII:c.

For best results, we have typically cultured FVIII:c-producing cells in media containing mammalian serum, e.g. conventional preparations of fetal bovine serum, in amounts of about 10% serum by volume relative to total media volume. (For the sake of simplicity, all serum concentrations hereinafter are expressed as a volume % of total media.) We have found that in the absence of such serum supplements both the yield and stability of the recombinant FVIII:c suffer significantly. However, the cost of serum supplements and the added inconvenience and expense in purification resulting from the use of serum rendered the use of serum an undesirable necessity and the wide scale use of recombinant FVIII:c a commercially less attractive alternative to natural FVIII:c purified from plasma.

After extensive experimental modifications of media for FVIII:c-producing cells we have surprisingly found a method for producing higher yields of stable FVIII:c-type proteins (as described hereinafter) using media containing reduced amounts of serum (e.g.,semi-defined media) or essentially serum-free media (defined media). We have found that host cells producing FVIII:c-type proteins produce recoverable, stable FVIII:c-type proteins in semi-defined and defined media in yields at least comparable to those obtained in the presence of 10% serum if the semi-defined or defined media contains a suitable amount of a hydrophobic substance such as VWF or certain phopholipids. We have further found that FVIII:c-type proteins produced in semi-defined or defined media lacking such supplements typically exhibit dramatic instability and are recoverable in extremely low yield if at all.

Evidence to date suggests either that VWF may have a stabilizing effect in vivo on the Factor VIII:c in plasma, or that the VWF can ellicit the in vivo release from storage depots or stimulate the in vivo synthesis and/or secretion of Factor VIII:c (Weiss, H. J. et al., 1977,J. Clin. Invest. 60: 390-404). It has also been suggested that thrombin-activated Factor VIII (derived from natural, human FVIII) is stabilized by

3

phospholipids, presumably with respect to thrombin-mediated degradation. See Andersson and Brown, 1981, Biochem. J. 200:161-167. However, to our knowledge there is no suggestion in the prior art as to any possible effect(s) of VWF or phospholipid on the in vitro production of Factor VIII:c-type proteins (where, for example, thrombin is substantially absent) or any suggestion of media supplements comprising VWF and/or phospholipids substantially free from the complex mixture of components present in mammalian serum in accordance with this invention.

Summary of the Invention

This invention concerns an improved method for the production of Factor VIII:c-type proteins.

"Factor VIII:c-type" proteins, as the term is used herein, means proteins exhibiting Factor VIII:c-type procoagulant activity. Factor VIII:c-type proteins within the ambit of this invention are encoded for by DNA sequences capable of hybridizing to DNA encoding Factor VIII:c. In addition to natural mammalian, e.g. human, Factor VIII:c, Factor VIII:c-type proteins include, for example, proteins which contain deletions of one or more amino acids between the 90 Kd and 69 Kd cleavage sites with respect to native Factor VIII:c, as described in greater detail in International Application No. PCT/US86/00774, published 23 October 1986. Factor VIII:c-type proteins also include Factor VIII:c analogs containing amino acid deletions between the 50/40 cleavage site and the 69 Kd cleavage site which may be produced by methods analogous to those disclosed in PCT/US86/00774. Factor VIII:c-type proteins further include analogs (with or without deletions as mentioned above) wherein one or more of the cleavage sites spanning arginine residues at positions 226, 336, 562, 740, 776, 1313, 1648 or 1721 have been rendered resistant to proteolytic cleavage, e.g., by replacement of one or more amino acids with different amino acids by conventional site-directed mutagenesis of the cDNA to be expressed. Factor VIII:c-type proteins thus include natural Factor VIII:c, "recombinant" Factor VIII:c and analogs thereof having procoagulant activity, and non-recombinant Factor VIII:c or analogs thereof produced by cell lines derived from cells which produce the protein.

The method of this invention thus utilizes mammalian cells which contain DNA encoding a Factor VIII:c-type protein and which are capable of expressing the protein. In accordance with the method of this invention the cells are cultured in media containing an effective amount of a stabilizing substance for a Factor VIII:c-type protein. Such substances include: (i) mammalian von Willebrand Factor (VWF), (ii) a stabilizing phospholipid or phospholipid mixture, and (iii) mixtures of VWF and phospholipid(s). Preferred effective amounts of VWF range from about 0.1-10 ug VWF/ml media, with 1-3 ug/ml being specially preferred. One readily obtainable source of suitable phospholipids comprises commercially available dry milk preparations such as dried skim milk and low-fat skim milk. Such dried milk preparations may be added to the media in amounts ranging from about 0.01% - 10% (weight of dry milk/volume of media). For optimal effect on Factor VIII production with minimal toxic effect on the cells, about 1% - 3% dry milk is presently preferred. The dry milk preparations may be conveniently sterilized by first preparing a 10% aqueous suspension of the milk and autoclaving. Another readily obtainable source of suitable phospholipids is commercially available soybean lecithin, which may be added to the medium in accordance with this invention preferably in liposome form.

"Phospholipid" as the term is used herein means an ester of phosphoric acid containing one or two molecules of fatty acid, an alcohol and a nitrogenous base. Examples of such phospholipids include Cephalin, phosphatidyl serine: phosphatidyl choline mixtures, phosphatidyl inositol, phosphatidyl ethanolamine, soybean lecithin and mixtures thereof, with soybean lecithin being especially preferred. Other phospholipids useful in this method as well as effective and/or optimal concentrations and/or mixtures thereof may be readily identified by those skilled in the art using methods described in greater detail hereinafter. Presently preferred effective amounts of phospholipid or phospholipid mixtures comprise about 1-1000 ug phospholipid or phospholipid mixture per ml of culture media, with concentrations greater than about 100 ug/ml being more preferred and concentrations between about 200-300 ug/ml being especially preferred. Additionally, it is presently preferred to add the phospholipid mixture to the culture media in the form of liposomes, preferably having a diameter of up to about 500 nm. Preferably the liposomes are unilamellar, although multilamellar liposomes may also be used. Most preferably the diameter of the liposomes is less than about 100 nm. Furthermore, liposomes made by conventional methods from said phospholipids may be used, either in admixture with or containing the Factor VIII:c-type protein, as a carrier or vehicle for administering the protein to patients. Where dried milk is used as the source of phospholipids, the dried milk may be added directly (rather than in the form of liposomes) to the media.

VWF may be obtained from mammalian, e.g., human, bovine, porcine, etc., serum by conventional methods or from cell lines derived from VWF-producing cells such as endothelial cells. Alternatively, "recombinant" VWF (i.e., VWF derived from genetically engineered cells) may be used. In one embodi-

4

ment, VWF-producing cells, such as cells suitably engineered to produce VWF, are cultured in the medium to condition it with VWF either prior to or simultaneously with the culturing of cells which produce a Factor VIII:c-type protein. Alternatively the recombinant VWF may be separately produced and added as an exogenous supplement to the media to be used for culturing the cells producing the Factor VIII:c-type protein. In another embodiment the cells which produce Factor VIII:c-type protein are suitably engineered, i.e. effectively transformed with a VWF transcription unit, such that the VWF and the Factor VIII:c-type protein are co-expressed by the same cells. In a further embodiment of this invention the media used for culturing the cells producing the Factor VIII:c-type protein contains both VWF, by virtue of one of the above-mentioned processes, and stabilizing phospholipid(s). In that case, it may be desirable to use reduced amounts of each component relative to the amounts used if used alone. By using appropriately supplemented defined media in accordance with this invention high levels of recoverable, stable Factor VIII:c-type activity are produced which may then be recovered and purified without the necessity for separation of serum components therefrom. The culture media used in this invention may additionally contain mammalian-derived serum, e.g. fetal bovine serum, preferably in amounts less than about 10%, more peferably in amounts less than about 5%, and even more preferably in amounts between 0 and 1%, although essentially serum-free media is especially preferred. Other conventional mammalian cell culture media supplements may also be added.

This invention is illustrated in the following examples which set forth typical procedures demonstrating, among other things, the ability to overcome the serum dependence in the production of recoverable, active Factor VIII:c-type proteins using phospholipids and VWF as media supplements. The examples are set forth to aid in an understanding of the invention but are not intended to, and should not be construed to, limit in any way the invention as set froth in the claims which follow thereafter.

EXAMPLE I

Establishment of Chinese Hamster Ovary Cell Lines which Express Human Factor VIII:c

The Factor VIII:c expression plasmid used in this Example was RxPy VIII-I which contains in clockwise order the polyomavirus enhancer, the first leader sequence of the adenovirus tripartite leader sequence, a Factor VIII:c transcription unit followed by a DHFR gene and SV40 polyadenylation signal, and a gene encoding tetracycline resistance. This plasmid was introduced into dihydrofolate reductase (DHFR) deficient Chinese hamster ovary cells by cotransformation with a DHFR expression plasmid and subsequent selection for cells that grow in the absence of added nucleotides. One particular pool of transformants designated lig 1 was subsequently grown in increasing concentrations of methotrexate (MTX) in order to amplify the DHFR and Factor VIII genes. The resultant cell line expressed high levels of Factor VIII activity as determined by either the ability to clot Factor VIII deficient plasma [Clotech (APPT) assay] or by the ability to generate Factor Xa in the presence of Factor IXa, phospholipid, calcium, and Factor X (Kabi Cotest assay). The ability of these CHO cells to produce Factor VIII:c is shown in Table I. The Factor VIII activity increased 10,000 fold with increasing levels of MTX resistance which correlated with the Factor VIII gene copy number. Other expression vectors may also be used in place of RxPy VIII-I so long as they are capable of directing the expression of Factor VIII:c or analogs thereof. Such vectors include, for example, pCVSVL2-VIII (ATCC No. 39813, see European Application No. 85202121.1) and pDGR-2 (ATCC No. 53100, see PCT/US86/00774-deletion analog). Other Factor VIII:c expression vectors may be prepared using conventional expression vectors and techniques containing, for example, the SalI fragment from pCVSVL2-VIII or pSP64-VIII (ATCC No. 39812). The SalI fragment from either vector contains a DNA sequence encoding full-length Factor VIII:c.

Table I

| Factor VIII Expression in Transfected and Amplified CHO cells | | |
|---|---|---|
| Pool | MTX (uM) | mU/ml/day of VIII:c |
| Lig 1 | 0.0 | 0.1 |
| | 0.02 | 11.5 |
| | 0.1 | 88.0 |
| | 1.0 | 288, 545* |
| | 5.0 | 644, 875* |
| | 20.0 | 1075 |

*Represents samples from two independent assays

Plasmids pAdD26SVpA(3) (Kaufman and Sharp, 1982, Mol. Cell. Biol.) and plasmid pRXPy-VIII-I were digested with Cla 1 and the resultant linearized DNA was ligated in vitro and coprecipitated with CaPO4 and used to transfect CHO DHFR deficient DUKX-BII cells. Cells which efficiently expressed DHFR would be expected to contain the enhancer element from pRXPyVIII-I associated with the DHFR gene from pAdD26SVpA(3). Results have been consistent with this hypothesis. Subsequent selection for increased DHFR expression by propagation of the cells in increasing concentrations of MTX results in cells which have amplified the Factor VIII gene and the DHFR gene. At each level of MTX selection, samples of the conditioned media (approximately $10^6$ cells/ml in alpha media supplemented with 10% fetal bovine serum) were taken for Factor VIII:c activity assay determined by the Kabi Coatest method modified to obtain sensitivity better than 0.05 mU/ml. Comparable results were also obtained by the one-stage activated partial thromboplastin time (APTT) coagulation assay using Factor VIII:c deficient plasma. All samples exhibited thrombin activation of 30 - 50 fold. For thrombin activation the samples were pretreated 1 -10 min with 0.2 units/ml thrombin at room temperature.

EXAMPLE II:Serum Dependence of Factor VIII:c Synthesis:

CHO cells (Lig 1 2 A subclone B10 in .1 uM MTX at 80% confluence) which are rinsed and fed with media containing 10% FCS or defined media (serum free, containing: 5 mg/ml BSA, insulin, transferrin, selenium, hydrocortisone and putrescine) accumulate Factor VIII activity. The rate of appearance in defined media is roughly 4-fold lower than in serum-containing media. The 4-fold difference becomes larger as the cells are propagated in the absence of serum. This results from inefficient rinsing of the cells. The rate of Factor VIII:c appearance increases fairly linearly up to 24 hrs. which suggests the VIII is stable in the media. This result is similar to results obtained with COS cells at lower levels of VIII expression (Approx. 10mU/ml/day) Cephalin, a mixture of phospholipids, can counteract at least part of the serum deficiency. CHO cells (Lig. 2 A pool in 20uM MTX) were separately cultured with and without serum and rinsed after 4 hr. The two CHO cultures were then split again, and a portion of the serum$^+$ and of the serum$^-$ CHO cells were supplemented with 5uM cephalin for an additional 2 hr. The other portion of the serum$^+$ and serum$^-$ CHO cells were not supplemented with cephalin. Media was assayed at 6 hrs. and 25 hrs. and results shown below:

| Conditions | | FVIII:c Activity* | |
|---|---|---|---|
| Serum | Cenhalin | 6 hr. | 25 hr. |
| +FCS | +Ceph | 594 | 1044 |
| -FCS | +Ceph | 408 | 514 |
| +FCS | -Ceph | 563 | 1492 |
| -FCS | -Ceph | 140 | 372 |

*mU/ml

The results suggest that cephalin alone can increase VIII activity in the absence of serum but that its effect is short lived (i.e. observed after 2 hrs. but is diminished at 25 hrs.). In one experiment the concentration of cephalin was increased and there was no further increase in VIII activity. This indicated some component in the cephalin was not rate limiting.

Part of the cephalin effect can be elicited by a simpler mixture of phospholipids or by single phospholipids. Cells (Lig 1 2 A .02 pool in 20uM MTX) were fed with 10% fetal calf serum or serum free media for 24 hrs. and then either cephalin (5um) or a mixture (1:4) of phosphatidyl serine and phosphatidyl choline (PCPS) were added to serum free cultures. Results from media assayed after 2 hrs. were:

| conditions | FVIII:c Activity |
|---|---|
| Serum Free | 110 |
| Serum Free + Cephalin | 489 |
| Serum Free + PCPS | 230 |
| 10% FCS | 613 |

This result demonstrates that phospholipids alone can increase VIII activity in conditioned media.

Analysis of the thrombin activability of VIII expressed in CHO cells growing under different conditions suggests that the presence of serum decreases the degree of thrombin activation. CHO cells (Lig 1 2 A pool in 20uM MTX) were rinsed and fed with media containing 10% fetal calf serum or defined media (5mg/ml BSA, transferrin, selenium, insulin, hydrocortisone, putrescine). 24 hrs. later either cephalin or 10% FCS was added and 2 hrs. later samples taken for assay and measure of thrombin activatibility:

| | | Assay at 26 hrs. | | |
|---|---|---|---|---|
| | | | | Coagulation Assay |
| Sample Media | Added at 24 h | Cobas mU/ml | mU/ml | (fold activation) |
| Defined media | - | 315 | 300 | 20X |
| Defined media | 5uM cephalin | 752 | 1040 | 34X |
| Defined media | 10% FCS | 684 | 440 | 8.4X |
| + 10% FCS | - | 1078 | 1200 | 10X |
| + 10% FCS | 5uM cephalin | 1154 | 1120 | 14X |
| Defined media | 10% boiled FCS | 543 | - | - |

These results indicate that the presence of serum increases the activity produced compared to serum free media but reduces the thrombin activatibility. In contrast, cephalin can compensate for the serum effect on increasing the activity of the VIII produced but does not reduce the thrombin activatibility. Thus, in serum free media, with the addition of cephalin 2 hrs. prior to harvest, CHO cells produce VIII at 1 unit/ml and this material exhibits a 34 fold thrombin activation. This experiment also demonstrates that 10% FCS added to serum free media 2 hrs. prior to assay can also increase the amounts of VIII activity. This ability was not dimimished by boiling the serum 10 min prior to its addition. Thus, the serum factor required for VIII activity is heat stable. We conclude that the serum factor required for increasing VIII may comprise two components: a phospholipid and another, heat stable factor which may be required to stabilize the phospholipid.

To determine whether the 10% serum was limiting for Factor VIII:c expression in the highly amplified CHO cell lines, we monitored the effect of increasing amounts of serum on the ability to elicit factor VIII:c activity in the cell line 10A1. 10A1 is a clone derived from selection of the Lig 1 pool for growth in 1 mM MTX. This experiment demonstrated the effect on Factor VIII activity of adding increasing amounts of fetal bovine serum to the 10A1 cells for 24 hrs. 50% serum yielded three-fold more activity in the 24 hr. conditioned media compared to 10% serum. Other results have indicated that the amount of active Factor VIII antigen is correspondingly increased when cells are propagated in 50% serum. Other cell lines, which express slightly lower levels of Factor VIII:c show less dramatic increases in Factor VIII:c activity upon growth in higher concentrations of serum. Thus there appears to be some limiting requirement for Factor VIII: expression in higher producing cells such as those which would be desirable for commercial-scale production of Factor VIII.

Example III

Serum Dependence of rFactor VIII:c Synthesis in Suspension Cultures of CHO.

The following table illustrates the dependency of recombinant Factor VIII:c (rFVIII) activity on serum levels in culture. A relatively low rFVIII producer, clone 1E6, was grown in suspension culture for 3 to 4 days in medium containing various concentrations of fetal bovine serum (FBS).

| Serum Concentration in Medium* | rVIII Activity (mU/ml) after 3-4 days | Average Productivity (U/$10^6$ cells/day) |
| --- | --- | --- |
| 10% FBS | 318 | 0.19 |
| 5% FBS | 100 | 0.03 |
| Defined* | 4 | 1/4-0.01 |

* RPMI 1640 was employed as basal medium for all of the above. The defined medium consists of insulin, 5ug/l; transferrin, 5ug/ml; serenium, 5ng/ml; hydrocortisone, $10^{-8}$ M, putrescene, 100ng/ml; BSA, 5mg/ml.

The same serum dependence has been observed with other rFVIII secreting CHO cell lines. These results do not reflect genetic instability since original expression levels can be regained on addition of serum.

We have found that addition of phospholipid to culture medium can replace the serum requirement, however relatively high concentrations of phospholipid are required (on the order of 10-20 fold higher than previously used with serum-containing media). With respect to the dependence of the recovery of FVIII activity on phospholipid concentration, we have found that supplementing defined media (DM) with 240 ug soybean lecithin (SL)/ml media yielded (after 24 hrs) about twice as much FVIII activity as was obtained in DM containing 160 ug SL/ml and five times as much FVIII activity as was obtained in DM containing 80 ug SL/ml. Nonetheless, DM containing 80 ug SL/ml provided measurably more FVIII activity than DM containing 1% Fetal Bovine Serum(FBS) (semi-defined media), while the semi-defined media provided significantly more FVIII activity than did DM alone. Significantly, the level of rFVIII generated over a 24h period in defined medium in the presence of 240 ug SL/ml media is at least as high as that generated in DM supplemented with 10% FBS. Increasing the concentration of SL above 240ug/ml resulted in no further increase in rVIII activity in this experiment. Illustrative results of one experiment are shown below:

| Media | rFVIII activity after 24h |
| --- | --- |
| Defined Media (DM) | ~ 40 mU/ml |
| DM + Soybean lecithin (SL) (240 ug SL/ml media) | ~ 270 mU/ml |
| Media containing Fetal bovine serum (FBS) (10% FBS) | ~ 200 mU/ml |

This data illustrates the increase in the rFVIII:c activity obtained with relatively high concentrations (e.g., 240 ug/ml) of soybean lecithin phospholipid in the absence of fetal bovine serum. CHO cells (1E6 in 0.1 umolar MTX) were suspended at a concentration of 3 x $10^5$ cells/ml in defined medium containing 5g/l of bovine serum albumin either in the absence or presence of phospholipid or medium containing 10% FBS for 24h at 37 C. At the conclusion of the incubation samples of cell-free conditioned medium were assayed for rFVIII:c activity by a chromogenic assay.

We have found that the addition of phospholipid in amounts up to about 320 ug/ml media causes no marked changed in cell growth in either defined or semi-defined media. In one set of experiments we found that maximum rFVIII activity was obtained in the culture where 320 ug phospholipid/mL media was added. In semi-defined medium, maximum levels were obtained after 72h where 240 ug/mL of soybean lecithin was added. These and other data illustrate that soybean lecithin added stepwise to cultures on days 0, 1, 2 and 3 allowed production of rFVIII. The optimum concentrations were around 240 ug/mL in these and other experiments regardless of the method of preparation of the phospholipid.

The cellular productivities in two different CHO cell lines from experiments similar to the above are shown below.

| Medium | Average Productivity (Cell Line - 1E6) (u/$10^6$ cells/day) |
|---|---|
| 10% FBS | 0.19 |
| 5% FBS | 0.03 |
| Defined | 1/4-0.01 |
| Defined + SL | 0.24 |
| Defined + 1% FBS + SL | 0.25 |

| Medium | Average Productivity* (Cell Line - H9.05) |
|---|---|
| 10% FBS | 0.43 |
| Defined + 1 % FBS + SL | 0.51 |

*Units as above

Thus, productivities of rFVIII from rCHO cells are at least equivalent in defined medium supplemented with phospholipid as in serum containing medium. However, as illustrated by the data above, the bulk quantity of rFVIII produced in defined medium is less than in serum containing medium. This is due to the fact that cells grow more rapidly and to higher cell densities in serum-containing medium rather than being more productive. On supplementation of defined medium with small quantities of serum (e.g. 1%) cell growth is improved. Indeed, after a short period of adaption cells will grow almost as well in semi-defined medium as in 10% FBS supplemented medium. We have found that rCHO cell lines (e.g., our H9.05) grow to similar cell densities and are at least equally productive (in Factor VIII) in phospholipid supplemented semi-defined medium as in 10% FBS supplemented medium.

Furthermore, we have also examined the physical nature of the soybean lecithin preparations. A size profile of a typical phospholipid preparation, where the soybean lecithin is Suspended in saline and passed three times through a Manton-Gaulin homogenizer, then filtered through a 0.2 um filter was obtained. The average mean size of the liposomes was around 100nm in diameter, suggesting that the majority of liposomes resulting from this process are small unilamellar vesicles (SUV's). The following experiment indicates that the size of the soybean lecithin liposomes may play an important role in the efficacy of the phospholipid, i.e., the ability of the SL to cause an increase in FVIII:c expression.

A soybean lecithin preparation was constituted in saline but not homogenized. The preparation contained significantly less SUV's than a normal (i.e., with homogenization) preparation (only 44% of the liposomes were below 100nms vs 74% in a normal prep). It also contained a significant population of multilamellar vesicles (MV's) which were around 500 nm's in diameter (30-40% of the total liposomes were MV's) which are present in only small quantities (usually < 5%) in normal preparations. The efficacy of this sample was reduced to about 60% of a normal sample indicating that the size of the liposomes may play an important role in the efficacy of the phospholipid.

Example IV

Porcine VWF can act to elicit Factor VIII:c activity from CHO cell propagated in the absence of serum.

Lig 1 (20uM MTX) cells were rinsed and fed defined media (alpha media containing insulin, transferrin, selenium, hydrocortisone, and putrescine, glutamine, and penicillin and streptomycin) added back with increasing concentrations of bovine serum albumin or with similar concentrations of ovalbumin Table II. Both proteins can act to elicit Factor VIII:c expression. However, when partially purified VWF is added back to media containing 5 g/l bovine serum albumin, the Factor VIII:c activity increased four-fold to even greater than the levels obtained upon propagation of the cells in 10% fetal bovine serum. This dramatically demonstrates the ability of VWF to elicit Factor VIII:c activity in the absence of serum. This result has been duplicated with different preparations of porcine VWF and also with purified human VWF.

In order to demonstrate that the ability to elicit factor VIII:c was due to VWF, the following experiment was performed. Cells which express Factor VIII:c were incubated in the presence of media containing serum derived from human VWF deficient plasma. Factor VIII:c activity in the CHO Lig 1 cells incubated in VWF deficient serum was 25% the level compared to normal human serum. When the porcine VWF preparation was added back to the VWF deficient serum, the Factor VIII activity increased to the 10% fetal bovine serum value. The effect could be elicited with as little as 2.50 ug/ml of VWF. See Table IIA. In another experiment, when the VWF concentration was decreased to 0.25 ug/ml, the activity was only 50% that of the 10% fetal bovine serum level.

The CHO cell line 1E6 which had been adapted over a 2-3 month period to grow in the absence of serum, in defined medium, was used in the following experiment in order to demonstrate that exogeneous VWF could increase the level of rVIII expression in defined medium in the absence of even residual amounts of bovine VWF. The data presented below shows that supplementation of defined medium with approximately 1 microgram/mL of porcine VWF allows expression of rFVIII equivalent to the level obtained in 10% fetal bovine serum supplemented medium. Since these cells had been grown for more than 3 months in the absence of FBS no trace bovine VWF was present. Thus the observed increase in rFVIII:c levels was likely due to exogeneous VWF.

| Effect of VWF on rFVIII Expression in the Absence of Serum | |
|---|---|
| MEDIA | rFVIII (mU/ml) |
| Defined Media (DM) | ~ 40 |
| DM + VWF | ~ 190 |
| Media containing FBS (10%) | ~ 200 |

This data illustrates the increase in the rFVIII activity by exogeneous, partially purified VWF (porcine) in the absence of fetal bovine serum. CHO cells (1E6 in 0.1 micromolar MTX) were suspended at a concentration of 3 x 10E5 cells/mL in defined medium containing 5g/L of bovine serum albumin either in the absence or presence of partially purified porcine VWF (at approximately 1 microgram/mL) or medium containing 10% FBS for 24h at 37 C. At the conclusion of the incubation samples of cell-free conditioned medium were assayed for rFVIII:c activity by chromogenic assay.

Table II: Factor VIII:c Expression in Defined Media with VWF Added back to Lig 1 Cells

| Defined Media + | | Units/ml/day |
|---|---|---|
| Ovalbumin (g/l) | 0 | 0.164 |
| | 0.5 | 0.189 |
| | 1.0 | 0.215 |
| | 2.0 | 0.280 |
| | 5.0 | 0.290 |
| | 20.0 | 0.380 |
| | 5.0 | |
| with procine VWF at 2.5 ug/ml | | 1.200 |
| | | |
| Defined Media + | | |
| Bovine Serum Albumin | | |
| (g/l) | 0 | 0.190 |
| | 0.5 | 0.320 |
| | 1.0 | 0.380 |
| | 2.0 | 0.375 |
| | 5.0 | 0.430 |
| | 20.0 | 0.490 |
| | 5.0 | |
| with porcine VWF at 2.5 ug/ml | | 1.350 |
| | | |
| 10% Fetal Bovine Serum | | 0.978, 1.075 |

## Table IIA:  Effect of VWF on Factor VIII Production

| MEDIA | Mu/ml/day |
|---|---|
| 10% fetal bovine serum | 1321 |
| defined media with 5 g/l bovine serum albumin | 342 |
| 10% normal human serum | 937 |
| 10% VWF deficient human serum | 246 |
| 10% VWF deficient human serum with porcine VWF added back at: | |
| 2.5 ug/ml | 1124 |
| 20 ug/ml | 1397 |

In order to examine the effect of added VWF on the amount of Factor VIII:c in the conditioned media, cells were labeled with a 1 hr. pulse of 35 - S methionine and chased in either media containing 10% fetal bovine serum, 10% VWF deficient human serum, or 10% VWF deficient human serum with porcine VWF added back. Results demonstrated that upon addition of VWF to VWF deficient serum, more Factor VIII:c (both the heavy 200kDa and the light 76kDa chains) was present in the media. No change in the intracellular synthesis of Factor VIII:c was observed. VWF addition to 10% fetal bovine serum resulted in no change in the level of Factor VIII:c in the conditioned media. These experiments indicate the VWF is necessary for the secretion and/or stability of Factor VIII:c.

EXAMPLE V

Expression of Human VWF in COS Cells

The cloning of a partial segment of the human VWF cDNA has previously been reported (Ginsberg, et al. 1985, Science). Subsequent to that report, the full length VWF cDNA has been assembled and its sequence determined. The cloning, sequence and expression of VWF have been described in detail in International Application No. PCT/US86/00760, published on 23 October 1986. We have inserted the full length cDNA clone into the expression vector pMT2 to produce pMT2-VWF (ATCC No. 67122). pMT2-VWF contains the adenovirus associated (VA) genes, SV40 origin of replication including the transcriptional enhancer, the adenovirus major late promoter including the adenovirus tripartite leader and a 5' splice site, a 3' splice site derived from an immunoglobulin gene, the VWF coding region, a non-coding DHFR insert, the SV40 early polyadenylation site, and the pBR322 sequences needed for propogation in E. coli. Details of this vector, which is a derivative of pQ2, are provided in Kaufman, Proc. Natl. Acad. Sci., USA 82:689-693 (1985). pMT2-VWF DNA was then prepared for COS cell transfection by conventional methods. Sixty hours after DEAE dextran mediated transfection of COS cells, the cells were labelled with 35-S methionine and media and cell extracts were immuneprecipitated with a rabbit anti-human polyclonal antibody (Calbiochem) and precipitates analyzed by SDS reducing gel electrophoresis. Results demonstrate a significant amount of VWF is synthesized in the transfected COS cells, the majority of its being secreted. In the conditioned media there is an approximately 260kDa protein and a 220kDa protein which resembles the completely processed form of VWF. Approximately 50% of the VWF synthesized is processed to the 200kDa form. When analyzed for multimer formation by non-reducing gel electrophoresis, it was found the VWF was associated into multimers, but not of extremely high molecular weight like those seen in plasma. The multimers ranged up to 1 million daltons by a rough estimate. Analysis of the VWF antigen in the COS cell conditioned media indicated the presence of human VWF at 0.35 ug/ml. Other analyses have indicated that the VWF expressed in COS cells specifically binds both platelets and collagen.

EXAMPLE VI

Recombinant VWF can elicit the expression of human Factor VIII:c.

The VWF expression plasmid pMT2-VWF was transfected onto COS cells by DEAE dextran mediated transfection and 36 hours post-transfection, the media changed to serum free (DMEM lacking serum). 72 hours later the COS cell conditioned media was harvested and applied to the CHO Lig 1 cells (20 uM MTX resistant) which were previously rinsed with serum-free media (at $10^6$ cells/ml). Twenty-four hours later the media was taken from the CHO cells and assayed for Factor VIII activity. The results are shown below and compared to Factor VIII:c activities from CHO cells propagated in 10% fetal bovine serum and in serum-free media for 24 hours.

| Media on CHO Lig 1 (20uM MTX) | mU/ml Factor VIII:c |
|---|---|
| Conditioned media from mock transfected COS cells | 141 |
| Conditioned media from VWF transfected COS cells* | 423 |
| 10% Fetal Bovine Serum | 950 |
| Serum-free media | 30 |

*The conditioned media in this experiment contained 0.3 ug/ml of human VWF.

Example VII: Introduction, Expression, and Amplification of VWF Genes in CHO Cells which express Factor VIII:c:

For expression of VWF in Chinese hamster ovary (CHO) cells, a second expression vector, pMT2ADA-VWF (ATCC #67172), was used with a protocol of selection for cells over-expressing the enzyme adenosine deaminase to amplify the plasmid sequences (Kaufman et al., 1986, Proc. Natl. Acad. Sci. 83:3136; U.S. Serial No. 619,801). A factor VIII:c expressing cell line which was cloned from lig1 2-a (from example I) in 1 mM MTX and designated 10A1, was used as recipient for transfer of pMT2ADA-VWF. pMT2ADA-VWF was introduced into 10A1 cells by protoplast fusion as described (Sandri-Goldin et al., 1981, Mol. Cell. Biol. 1:743). E. coli DH5 cells harboring pMT2ADA-VWF (DH5 was used to minimize homologous recombination and deletion of the VWF sequences) were grown in 50 ml of L-broth containing 50 ug/ml ampicillin to an $A_{600}$ of 0.6. Chloramphenicol was added to 250 ug/ml and the culture incubated at 37 C for an additional 16 hrs, in order to amplify the plasmid copy number. A suspension of protoplasts was prepared as described (Sandri-Goldin et al., 1981), added to 10A1 cells at a ratio of approximately 1-2 x $10^4$ protoplasts/cell, and centrifuged onto the cells at 2000 rpm for 8 minutes in an IEC model K centrifuge. After centrifugation, the supernatant was removed by aspiration and 2 ml of polyethylene glycol solution (50g of PEG 1450, Baker Chem. Co., in 50 ml of Dulbecco's modified medium) was added to each plate. Cells were centrifuged again at 2000 rpm for 90 seconds, the polyethylene glycol solution removed, and the plates rinsed 3 times in alpha medium containing 10% (v/v) fetal calf serum. Cells were then plated into tissue culture dishes in medium containing 100 ug/ml kanamycin, 10 ug/ml each of penicillin and streptomycin, and 20 uM MTX. Two days later the cells were trpysinized and subcultured 1:15 into ADA selective media with 10% dialyzed fetal calf serum, .1um deoxycoformycin, 10 ug/ml of penicillin and streptomycin, and in the presence and absence of 20 uM MTX. The ADA selective media (AAU) contained 1.1 mM adenosine, 10 ug/ml alanosine and 1mM uridine. Subsequently it was shown that removal of the MTX selection at this stage resulted in a decrease in the factor VIII:c expression. Subsequently, the MTX has been left in the ADA selective media.

It was possible to amplify the VWF gene by selection for growth in increasing concentrations of 2'-deoxycoformycin (dCF) in the presence of cytotoxic concentrations of adenosine. A pool of transformants (6 colonies) was prepared from 10A1 cells 6 colonies in pool selected for ADA in the presence of 20 uM MTX. The ADA selection mean was changed by sequentially increasing the concentration of 2'deoxycoformycin (steps of 0.1 uM, 0.5 uM, 1.0 uM and 2.0 uM) in the presence of 20 uM MTX. At each step, the production of VWF and of factor VIII:c was measured after 24 hours in the presence of 10% fetal calf serum (FCS) or in defined media. The results are summarized below:

13

| Coxpression of VWF and FVIII:c in CHO cell lines | | | | | |
|---|---|---|---|---|---|
| Cell line | Selection | | VWF Antigen | | Factor VIII:c uUnits/cell |
| | dCF uM | MTX uM | ug/ml | pg/cell | |
| 10A1 (no VWF) | | | | | .38*<br>0.93** |
| 10A13 a pool | 0.1<br>0.5<br><br>1.0<br><br>2.0 | 20<br>20<br><br>20<br><br>1000 | 0.07<br>0.8<br><br>24<br><br>7.4 | 0.1<br>1.14<br><br>30<br><br>24 | <br>0. 63*<br>0. 89**<br>0. 63*<br>1.1**<br>1.4*<br>1.5* |

\* = in defined media;

\*\* = in media containing 10% Fetal calf serum; VWF antigen was determined by an ELISA assay using affinity-purified rabbit-anti-VWF antiserum (Calbiochem Behring, 782301), purified VWF antigen from normal human plasma pools to serve as standards and controls, and IgG isolated from Calbiochem-Behring, 782301, and conjugated with alkaline phosphatase. Factor VIII:c activity was determined by the chromogenic assay described in Example I.

These results demonstrate that VWF expression increased with increasing ADA selection. In addition, expression of factor VIII:c was not dependent on the presence of serum, as observed by line 10A13-a in 2 uM dCF and 1000 uM MTX which expresses 1.4 uUnits/cell/day of factor VIII:c in defined media.

Example VIII : Fusion of CHO cells expressing Factor VIII:c and CHO cells expressing VWF:

The VWF gene has been introduced into CHO DHFR deficient cells (DUX-B11, Chasin and Urlaub, 1980, Proc. Natl. Acad. Sci. 77:44216). Two approaches have been taken in order to obtain cells that express either MTX resistance or dCF resistance associated with VWF expression. Then either cell line can be subsequently used to fuse to other cells that express factor VIII:c with the ability to select for either MTX or dCF resistance.

MTX Amplification in CHO DHFR deficient Cells

Plasmid pMT2VWF and pAdD26SVpa(3) were mixed 10:1 and transfected by CaPO$_4$ coprecipitation into CHO DUKX-B11 cells as described by Kaufman and Sharp (1982, J. Mol. Biol. 150:601). Cells were selected for the DHFR positive phenotype by growth in the absence of nucleosides and colonies pooled and selected for increasing MTX resistance. The results indicated that VWF expression increased with increasing MTX resistance and are depicted in the Table below:

| Selection | ng/ml VWF |
|---|---|
| 0.02 uM MTX | - - - - |
| 0.2 uM MTX | 56 |
| 1.0 uM MTX | 91 |
| 5.0 uM MTX | 278 |

dCF Selection for VWF in CHO DHFR Deficient Cells

The plasmid pMT2ADA-VWF was introduced into CHO DUKX-B11 cells as described in Example VIII and cells selected for growth in ADA selective alpha media with 4 uM xyl-A, 0.03 uM dCF, 10 ug/ml hypoxanthine, 10 ug/ml thymidine, and 10 ug/ml of penicillin and streptomycin. One clone PM5F was derived which expressed 3-5 pg of VWF/cell/day. This clone was subsequently used for fusion to factor VIII:c cell lines and as a recipient for the introduction of factor VIII:c genes.

Fusion of Factor VIII:c and VWF Expressing Cell Lines

The factor VIII:c expression plasmid pLA2 has been described (Toole et al., 1986, Proc. natl. Acad.; International Application No. PCT/US86/00774). This plasmid has been introduced into DUKX-B11 CHO cells by protoplast fusion with selection for DHFR from the 3' region of the factor VIII-DHFR transcript (See PCT/US86/00774). A cell line was derived by selection for MTX resistance to 1.0 uM MTX and has been named LA3-5. This cell line expresses a deleted form of Factor VIII:c at 3-5 uUnits/cell/day (in 10% fetal calf serum). This modified factor VIII:c also binds to and requires VWF for efficient synthesis. LA3-5 was fused to PM5F and hybrids were selected that expressed both the MTX resistance and dCF resistance phenotypes.

For fusion, PM5F was treated with diethylepyrocarbonate (0. 03% for 30 minutes on ice) in order to kill the PM5F. These cells were then fused by polyethylene glycol-induced cell fusion to LA3-5: DEPC treated pMSF cells were centrifuged onto LA 3-5 (1.5 x $10^6$ cells) at 2000 rpm for 8 minutes in an IEC model K centrifuge. After centrifugation, supernatant was removed and 2 ml of 50% PEG solution was added. PEG was left on for 45 seconds and cells were washed thoroughly with serum free medium. Cells were left plated with medium containing serum for 48 hrs. and were then subcultured into selective medium containing 4uM xyl-A, 0.03 uMdCF, in the presence of 10 ug/ml of each of the following: thymidine, hypoxanthinine, streptomycin, and penicillin. However, it was not necessary to include the thymidine and hypoxanthine. A pool of hybrids was obtained which expressed 0.73 pg/cell/day of VWF and 0.2 - 2.0 units/ml/day of factor VIII:c. The pool was subsequently grown in the absence of thymidine and hypoxanthine in the presence of 0.5 uM MTX. These cells were cloned in alpha media with 4 uM xyl-A, 0.03 uM dCF, and 0.5 uM MTX to obtain the following clones:

| Factor VIII:c and VWF Coexpression in CHO Cells | | |
|---|---|---|
| Clone | VWF Expression (pg/cell) | Factor VIII: c Expression (uUnits/Cell-media) |
| E6 | 16 | 2.8 - defined<br>3.8 -10% FCS |
| B9 | 20 | 4.5 - defined<br>5.1 - 10% FCS |
| H6 | 34 | 7.7 - defined<br>8.7 - 10% FCS |
| B1 | 8. | 10.5 defined<br>11.8 10% FCS |

These results demonstrate the ability of the VWF and Factor VIII:c co-expressing cell lines to produce high levels of factor VIII:c in defined media.

Introduction or Factor VIII:c Genes into Cells Expressing VWF

A factor VIII:c deletion mutant of 907 amino acids has been constructed by heteroduplex mutagenesis (PCT/US86/00774) which directly fuses the 90 kDa cleavage site (at residue 740) to the 76 kDa cleavage site (at 1647). The resultant plasmid p90-76R has the factor VIII:c coding region 5' on the polycistronic transcription unit in pMT2. Protoplasts of E. coli HB101 harboring this plasmid were prepared and fused to the VWF expressing cell line PM5F as described in Example VIII. 48 hrs after recovery from protoplast fusion, the cells were subcultured into DHFR selection media (alpha media lacking nucleosides with 10% dialyzed fetal calf serum, 4uM xyl-A, and 0.03 uM dCF. After two weeks, transformants were isolated and assayed for Factor VIII:c expression. Approximately 20% of the transformants which had arisen expressed both VWF and Factor VIII:c. Results for one transformant are indicated below:

# EP 0 253 870 B1

| Cell Line | Factor VIII:c Activity | |
|---|---|---|
| F1 | 1.5 uUnits/cell<br>0.95 uUnits/cell | 1375 mUnits/ml def. media<br>1330 mUnits/ml 10% FCS |
| (with VWF = 1.69 ug/ml, 1.85 pg/cell) | | |

These results demonstrate the ability to select for the DHFR phenotype in the PM5F cell line and to coexpress factor VIII:c and the VWF in order to alleviate the serum dependence for factor VIII:c expression.

## Claims

1. A method for the production of a recombinant Factor VIII:c-type protein comprising the steps of:
   a) transfecting mammalian cells with a gene encoding a Factor VIII:c-type protein;
   b) culturing the cells in a serum-free culture medium conditioned with a composition comprising von Willebrand Factor (vWF), a phospholipid or a mixture of the two;
   c) recovering the Factor VIII:c-type protein from the culture medium.

2. The method of claim 1, in which the phospholipid is a phospholipid mixture.

3. A method according to claim 1 or claim 2, wherein the vWF comprises a naturally-produced mammalian vWF.

4. A method according to claim 1 or claim 2, wherein the vWF comprises a recombinant mammalian vWF.

5. A method according to any of claims 1, 2 or 4, wherein the mammalian cells are cocultured with recombinant mammalian cells expressing a recombinant vWF gene.

6. A method according to any of claims 1, 2 or 4, further comprising the steps of transfecting the mammalian cells with a gene encoding vWF and culturing the cells to coexpress vWF and the Factor VIII-c-type protein.

7. A method according to any preceding claim, wherein the phospholipid is selected from cephalin, phosphatidyl serine, phosphatidyl choline, phosphatidyl inositol, phosphatidyl ethanolamine, soy bean lecithin or a mixture thereof.

8. A method according to any preceding claim, wherein the phospholipid is in the form of dried milk.

9. A method according to any preceding claim, further comprising the step of amplifying the gene encoding the Factor VIII:c-type protein.

10. A method according to any one of claims 5 to 9, further comprising the step of amplifying the gene encoding vWF.

11. A method according to any preceding claim, wherein the Factor VIII:c-type protein recovered from the culture medium is associated with vWF.

12. A method according to claim 11, further comprising the step of separating the Factor VIII:c-type protein from the vWF.

## Patentansprüche

1. Verfahren zur Herstellung eines rekombinanten Faktor VIII:c-Typ Proteins, welches Verfahren folgende Schritte umfaßt:
   a) Transfektion von Säugetierzellen mit einem Gen, das für ein Faktor VIII:c-Typ Protein codiert;
   b) Züchtung der Zellen in einem serumfreien Kulturmedium, das mit einer Zusammensetzung konditioniert ist, die von Willebrand-Faktor (vWF), ein Phospholipid oder eine Mischung der beiden enthält;

16

EP 0 253 870 B1

c) Gewinnung des Faktor VIII:c-Typ Proteins aus dem Kulturmedium.

2. Verfahren nach Anspruch 1, bei welchem das Phospholipid eine Phospholipidmischung ist.

3. Verfahren nach Anspruch 1 oder 2, bei welchem der vWF einen auf natürliche Weise produzierten Säugetier-vWF umfaßt.

4. Verfahren nach Anspruch 1 oder 2, bei welchem der vWF einen rekombinanten Säugetier-vWF umfaßt.

5. Verfahren nach einem der Ansprüche 1, 2 oder 4, bei welchem die Säugetierzellen mit rekombinanten, ein rekombinantes vWF-Gen exprimierenden Säugetierzellen co-gezüchtet werden.

6. Verfahren nach einem der Ansprüche 1, 2 oder 4, das weiters die Schritte der Transfektion der Säugetierzellen mit einem für vWF codierenden Gen und die Züchtung der Zellen zur Co-Expression des vWF und des Faktor VIII:c-Typ Proteins umfaßt.

7. Verfahren nach einem der vorhergehenden Ansprüche, bei welchem das Phospholipid ausgewählt ist aus Cephalin, Phosphatidyl-Serin, Phosphatidyl-Cholin, Phosphatidyl-Inositol, Phosphatidyl-Ethanolamin, Sojabohnen-Lecithin oder einer Mischung derselben.

8. Verfahren nach einem der vorhergehenden Ansprüche, bei welchem das Phospholipid in Form getrockneter Milch eingesetzt wird.

9. Verfahren nach einem der vorhergehenden Ansprüche, das weiters den Schritt der Amplifizierung des für das Faktor VIII:c-Typ Protein codierenden Gens umfaßt.

10. Verfahren nach einem der Ansprüche 5 bis 9, das weiters den Schritt der Amplifizierung des für vWF codierenden Gens umfaßt.

11. Verfahren nach einem der vorhergehenden Ansprüche, bei welchem das Faktor VIII:c-Typ Protein, das aus dem Kulturmedium gewonnen wird, mit vWF assoziiert ist.

12. Verfahren nach Anspruch 11, das weiters den Schritt der Abtrennung des Faktor VIII:c-Typ Proteins von dem vWF umfaßt.

**Revendications**

1. Procédé pour la production d'une protéine recombinante de type facteur VIII:c comprenant les étapes consistant à:
   (a) transfecter des cellules de mammifère avec un gène encodant une protéine de type facteur VIII:c:
   (b) cultiver les cellules dans un milieu de culture exempt de sérum conditionné avec une composition comprenant le facteur de von Willebrand (vWF), un phospholipide ou un mélange des deux;
   (c) récupérer la protéine de type facteur VIII:c d'avec le milieu de culture.

2. Procédé selon la revendication 1, dans lequel le phospholipide est un mélange de phospholipides.

3. Procédé selon la revendication 1 ou 2, dans lequel le vWF comprend un vWF de mammifère produit naturellement.

4. Procédé selon la revendication 1 ou 2, dans lequel le vWF comprend un vWF recombinant de mammifère.

5. Procédé selon l'une quelconque des revendications 1, 2 ou 4, dans lequel les cellules de mammifère sont co-cultivées avec des cellules recombinantes de mammifère exprimant un gène vWF recombinant.

6. Procédé selon l'une quelconque des revendications 1, 2 ou 4, comprenant de plus les étapes consistant à transfecter des cellules de mammifère avec un gène encodant le vWF et à cultiver les

17

cellules pour co-exprimer le vWF et la protéine de type facteur VIII:c.

7. Procédé selon l'une quelconque des revendications précédentes, dans lequel le phospholipide est choisi parmi la céphaline, la phosphatidylsérine, la phosphatidylcholine, le phosphatidylinositol, la phosphatidyléthanolamine, la lécithine de soja ou un mélange de ceux-ci.

8. Procédé selon l'une quelconque des revendications précédentes, dans lequel le phospholipide est sous la forme de lait séché.

9. Procédé selon l'une quelconque des revendications précédentes, comprenant de plus l'étape d'amplification du gène encodant la protéine de type facteur VIII:c.

10. Procédé selon l'une quelconque des revendications 5 à 9, comprenant de plus l'étape d'amplification du gène encodant le vWF.

11. Procédé selon l'une quelconque des revendications précédentes, dans lequel la protéine de type facteur VIII:c, récupérée à partir du milieu de culture, est associée au vWF.

12. Procédé selon la revendication 11, comprenant de plus l'étape de séparation de la protéine de type facteur VIII:c d'avec le vWF.